# EUROPEAN PATENT SPECIFICATION

(11) **EP 3 140 761 B1**
(45) Date of publication and mention of the grant of the patent: **03.07.2024**
(21) Application number: 15789939.4
(22) Date of filing: 05.05.2015
(51) Int. Cl.: G16H 40/40

(54) **INTELLIGENT SERVICE ASSISTANT - INSTRUMENT SIDE SOFTWARE CLIENT**
INTELLIGENTER DIENSTASSISTENT - INSTRUMENTENSEITIGER SOFTWARE-CLIENT
ASSISTANT DE SERVICE INTELLIGENT - CLIENT LOGICIEL CÔTÉ INSTRUMENT

(30) Priority: 07.05.2014 US 201461989633 P
(43) Date of publication of application: 15.03.2017
(73) Proprietor: Siemens Healthcare Diagnostics Inc., Tarrytown, NY 10591 (US)
(72) Inventor: RUDORFER, Arnold, Princeton, New Jersey 08540 (US); TUDOSE, Catalin, Yorktown Heights, New York 10598 (US); SAWANT, Milind, Newark, DE 19711 (US); MAGOWAN, Steve, Elkton, Maryland 21921 (US)
(74) Representative: Schweitzer, Klaus
(86) International application number: PCT/US2015/029197
(87) International publication number: WO 2015/171581

(56) References cited:
- US-A1- 2007 198 213
- US-A1- 2009 275 805
- US-A1- 2010 295 685
- US-A1- 2011 235 873
- US-B1- 6 473 659
- US-B1- 6 473 659

## Description

### CROSS-REFERENCE TO RELATED APPLICATIONS

This application claims priority to U.S. provisional application Serial No. 61/989633 filed May 7, 2014.

### TECHNOLOGY FIELD

Embodiments of the present invention relates generally to servicing in vitro diagnostics (IVD) instruments, and more particularly, to systems and methods for troubleshooting issues related to in vitro diagnostic instruments that generate analytical test results from a patient's bodily fluids.

### BACKGROUND

IVD allows laboratories to assist in the diagnosis of disease based on assays performed on patient fluid samples. IVD includes various types of analytical tests and assays typically conducted with automated analyzer systems (e.g. clinical chemistry, immunoassay, analyzers) onto which fluid containers, such as tubes or vials containing patient samples (e.g., blood samples), have been loaded. The analyzer system(s) extracts a fluid sample from the vial and combines the sample with various reagent fluids (reagents) in special reaction cuvettes or tubes (referred to generally as reaction vessels).

IVD instruments are typically serviced at regular intervals for proper functioning to deliver tests to labs and doctors. When non-scheduled instrument issues occur, instrument uptime, operator workflow and efficiency are negatively affected.

Conventional methods for troubleshooting IVD instrument issues are complex (e.g., identification of root causes of issues) and time consuming (e.g. time to determine correct path for a solution). These conventional methods rely on operator knowledge to fix the issues, thereby increasing operator workflow. Some conventional methods for troubleshooting IVD instruments issues include identifying the issues through an error condition or error code. In these conventional methods, an operator may look up how to troubleshoot an issue in a printed manual based on the error condition or error code. Operators may also search online for troubleshooting issues. Other conventional methods for troubleshooting IVD instrument issues include identifying the issues through a representative via the telephone or online. These conventional methods which include defining an issue and identifying solutions/strategies are time consuming and inefficient.

US 2007/198213 A1 discloses systems and methods for guiding and managing laboratory analytical process control operations and biometric quality control (QC). It concerns QC error flags, different causes for them and troubleshooting of the errors. US 2007/198213 A1 does not disclose the in vitro diagnostic instrument comprising a database, a user interface and a computing device, does not disclose the user interface being used to prompt an operator to indicate one or more of the corrective actions displayed in the ordered list to successfully resolve the identified issue, nor does it disclose storing at a second database the one or more corrective actions determined to successfully resolve the issue at the in vitro diagnostics instrument and the corresponding corrective actions determined to successfully resolve the one or more issues at the other in vitro: diagnostics instruments.

### SUMMARY

Systems and methods are described herein which relate generally to servicing in vitro diagnostics (IVD) instruments, and more particularly, to systems and methods for troubleshooting issues related to in vitro diagnostic instruments that generate analytical test results from a patient's bodily fluids. The scope of the invention is defined by the appended claims 1-8.

The present invention is directed to a method of providing troubleshooting for an IVD instrument according to claim 1. For example, in one embodiment, the issue may be software-related, hardware-related, or assay-related. The issue may be identified, for example, by way of an issue code generated by software associated with the portion of the instrument, and/or a selection of parameters related to the portion of the instrument (e.g., issue category, system module, available method, usage category, etc.). To resolve the issue, an ordered list of one or more corrective actions is provided via a user interface in communication with the computing device. The ordered list is stored in a database and the computing device receives an indication via the user interface of the one or more corrective actions determined to successfully resolve the issue. The computing device is in communication with one or more other computing devices configured to receive indications of corresponding corrective actions determined to successfully resolve the issue at other instruments and provide the corresponding corrective actions to the computing device.

In some embodiments, the aforementioned method further comprises creating a report comprising (i) the one or more corrective actions determined to successfully resolve the issue and (ii) a navigation path indicating one or more steps used to provide the one or more corrective actions determined to successfully resolve the issue.

The method further comprises providing, via a network, the corrective actions determined to successfully resolve the issue to a second computing device. This second computing device is in communication with (i) the computing device and (ii) one or more other computing devices configured to receive indications of corresponding corrective actions determined to successfully resolve one or more of the issues at other IVD instruments and provide the corresponding corrective actions to the second computing device. Additionally, data is stored at a second database in communication with the second computing device corresponding to the one or more corrective actions determined to successfully resolve the issue at the IVD instrument and the corresponding corrective actions determined to successfully resolve the one or more issues at the other IVD instruments.

In some embodiments, the aforementioned method further includes associating, by the second computing device, a plurality of corrective actions with corresponding issues and determining, by the second computing device, updated ordered lists of the one or more corrective actions for each corresponding issue based on at least one of: (i) a probability of the one or more corrective actions to successfully solve each corresponding issue; (ii) a success rate of the one or more corrective actions indicated at each of the instruments; and (iii) a priority. The ordering of the one or more corrective actions for solving each corresponding issue may then be stored at the second database. In one embodiment, one or more of the corrective actions and the corresponding issues associated with the one or more corrective actions are stored at the database. One of the determined updated ordered lists of the corrective actions for solving the issue are received from the second database and stored at the database in communication with the computing device, the updated ordered list. The updated ordered list may then be provided via the user interface in communication with the computing device. In one embodiment, the information provided to the second database and updates to the second database are provided through a second user interface of the second computing device.

Further, the present invention is directed to a system for providing troubleshooting for an IVD instrument according to claim 8. The system includes the IVD instrument, and the IVD instrument comprises a database, a user interface, and a computing device. The database is configured to store a plurality of issues each associated with a portion of the instrument and a plurality of corrective actions for resolving corresponding issues. For example, in some embodiments, the issue is one of software-related, hardware-related, or assay-related. The user interface comprises one or more display elements configured to provide user interaction to identify an issue of the issues associated with a portion of the instrument. For example, in some embodiments, the issue is identified through one or more of (i) an issue code generated by software associated with the portion of the instrument, and (ii) a selection of parameters related to the portion of the instrument, the selection via the user interface. The selected parameters may include, for example, an issue category, a system module, an available method, and/or a usage category. The computing device included in the system is configured to receive the identification of the issue and to determine one or more of the corrective actions for resolving the issue. The user interface is configured to further display an ordered list of the one or more corrective actions for resolving the issue. This list may be ordered, for example, based on one or more of priority and success rate. The user interface is further configured to receive an indication of the one or more corrective actions that successfully resolved the issue.

The system further comprises a central computing device and a central database. In these embodiments, the computing device is further configured to transmit a report comprising the one or more corrective actions that successfully resolved the issue to the central computing device, the central computing device configured to analyze the report and a plurality of reports from corresponding in vitro diagnostic instruments. The analysis by the central computing device may include, for example, associating issues with corrective actions; and determining an ordering of corrective actions for each issue, the ordering based on one or more of priority and success rate. The central database included in the system is configured to store the report and the reports. In some embodiments, the report includes a navigation path indicating steps taken resulting in the corrective actions that resolved the issue. In some embodiments, the database associated with the computing device maintains issues and associated listings of corrective actions, and receives updates thereto from the central database. In some embodiments, inputs and updates to the central database are provided through a user interface of the central computing device.

The system for providing troubleshooting for an IVD instrument includes a database, a user interface, a computing device, and a central computing device. The database is configured to store issues, with each issue being associated with a portion of the instrument and corrective actions for resolving corresponding issues. The user interface includes display elements which are configured to: provide user interaction to identify an issue of the issues associated with a portion of the instrument; display an ordered list of corrective actions for resolving the issue; and receive an indication of the corrective actions that successfully resolved the issue. The computing device is in communication with the user interface configured to receive the identification of the issue and to determine the one or more corrective actions for resolving the issue. The central computing device is in communication with the computing device and other computing devices in communication with other instruments. This central computing device is configured to analyze the one or more corrective actions that successfully resolved the issue and other corrective actions that successfully resolved the issue at the other instruments and provide an updated ordered list to the computing device to be displayed at the user interface.

Additional features and advantages of this disclosure will be made apparent from the following detailed description of illustrative embodiments that proceeds with reference to the accompanying drawings.

### BRIEF DESCRIPTION OF THE DRAWINGS

The foregoing and other aspects of the embodiments disclosed herein are best understood from the following detailed description when read in connection with the accompanying drawings. For the purpose of illustrating the embodiments disclosed herein, there is shown in the drawings embodiments that are presently preferred, it being understood, however, that the embodiments disclosed herein are not limited to the specific instrumentalities disclosed. Included in the drawings are the following Figures:
FIG. 1 is a system diagram of an exemplary system of providing troubleshooting for an in vitro diagnostics instrument according to embodiments disclosed herein;
FIG. 2 is a block diagram illustrating an exemplary instrument of the system shown in FIG. 1;
FIG. 3 is an illustration of an exemplary configuration of a communication architecture that may be used with embodiments disclosed herein;
FIG. 4 is a screen shot illustrating an exemplary identification of an instrument issue according to an embodiment;
FIG. 5 is a screen shot illustrating an exemplary ordered list of corrective actions displayed to resolve the identified issue shown in FIG. 4;
FIG. 6 is a screen shot illustrating an exemplary prompt to a user to indicate one or more corrective actions to successfully resolve the identified issue shown in FIG. 4;
FIG. 7 is a screen shot illustrating an exemplary updated list that is ordered based on a probability of the corrective actions to successfully solve the issue shown in FIG. 4; and
FIG. 8 illustrates an example of a computing environment within which embodiments of the invention may be implemented.

### DESCRIPTION OF EXEMPLARY EMBODIMENTS

Embodiments include systems and methods that provide an ordered list of corrective actions for solving an issue associated with a portion of an instrument. In some embodiments, the ordered list of corrective actions is provided based on a probability of success of the corrective actions used across a population of the instruments.

Embodiments provide well defined consistent structure for complex issue/solution strategies by using success rates of previously determined corrective actions. Embodiments provide corrective actions for different portions of instruments (e.g., mechatronic system, system architecture, software components), different conditions (e.g., environmental conditions such as temperature and humidity) and different parameters (e.g., usage or actual volume of tests such as throughput, types of samples (such as whole blood, urine, serum, or plasma) and operator history (such as history of performing routine maintenance as prescribed by the instrument manufacturer). Embodiments utilize one or more databases (e.g., central database in communication with a plurality of instruments) that include online solution strategies based on feedback (e.g., customer service knowledge, corrective actions and strategies) from other operators or instruments addressing issues under various conditions and variables.

Embodiments provide an intelligent service assistant (ISA) that enables an operator to resolve system hardware, software, and assay issues. The ISA on the client side receives customer service knowledge, via a plurality of instruments, for troubleshooting through a software application providing online solution strategies.

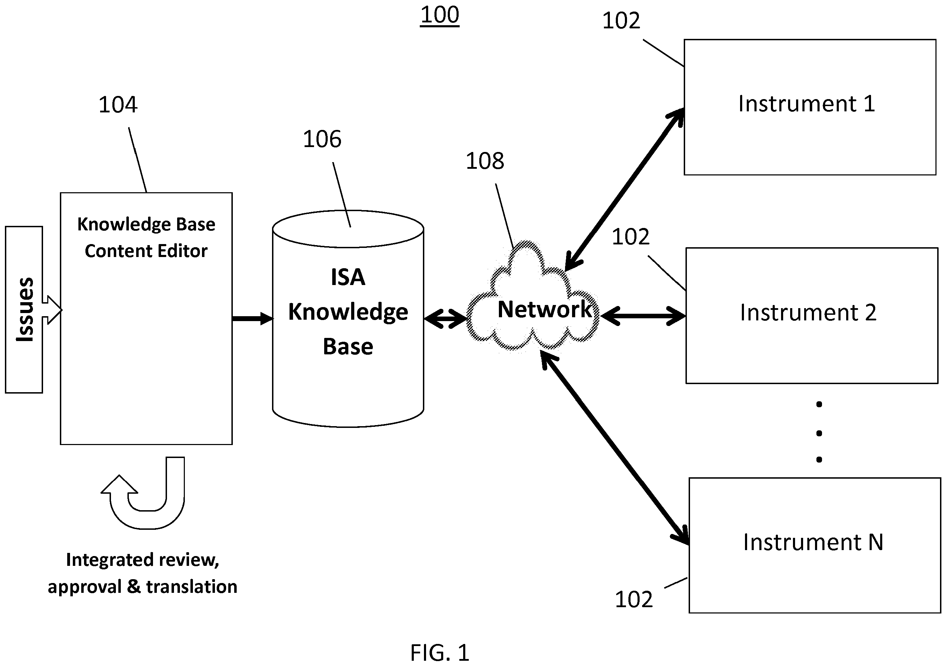

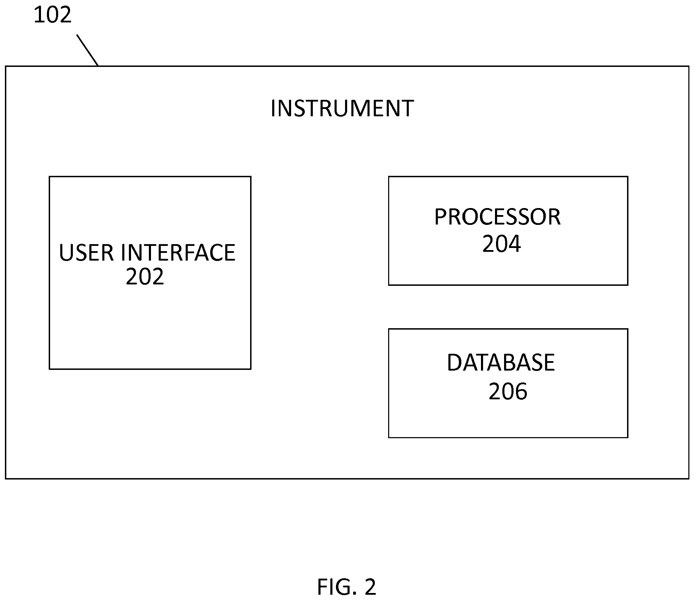

FIG. 1 is a system diagram of an exemplary ISA system 100 of providing troubleshooting for an in vitro diagnostics instrument 102 according to embodiments disclosed herein. FIG. 2 is a block diagram illustrating an exemplary instrument 102 of ISA system 100 shown in FIG. 1. Referring generally to FIG. 1 and FIG. 2, the system 100 includes instruments 102. Embodiments may include ISA systems having any number of instruments. Each instrument may have a database dedicated to itself. For example, as shown in FIG. 2, an instrument 102 include a dedicated database 206. Database 206 is configured to store a plurality of issues each associated with a portion of the instrument 102 and a plurality of corrective actions for resolving corresponding issues. The instrument 102 also includes a user interface 202 (e.g., a graphical user interface (GUI)) comprising one or more display elements configured to provide user interaction to identify an issue of the plurality of issues associated with a portion of the instrument 102. The instrument 102 includes a computing device, such as processor 204 dedicated to an instrument 102, in communication with the user interface 202. The processor 204 is configured to receive the identification of the issue and to determine one or more of the plurality of corrective actions for resolving the issue. The user interface 202 is configured to display an ordered list of the one or more corrective actions for resolving the issue and receive an indication of the one or more corrective actions that successfully resolved the issue.

As shown in FIG. 1, the system 100 also includes a second computing device, such as a knowledge base content editor 104, and a second database 106 in communication with the knowledge base content editor 104. In some aspects, the second computing device 104 may be a central computing device in communication with each of the instruments 102. In some aspects, the second database 106 may be a central database in communication with each of the instruments 102. For example, as shown in FIG. 1, the second computing device 104 and the second database 106 are in communication with each of the instruments 102 via network 108.

The ISA knowledge base 106 may include identified issues with related solution strategies. Various users, such as development engineers and customer service engineers may access the knowledge base content editor 104 to create and/or edit issues that are encountered, carry out a root cause analysis, and derive applicable solution strategies for remedying the issue. Each issue, with its set of solution strategies, may be stored in the ISA knowledge base 106.

Embodiments may include any number of computing devices and any number of data bases. For example, instruments 102 may include subsets of instruments with the instruments of each subset being in communication with a corresponding communication device. Each communication device in communication with the instruments of each subset may, in turn, be in communication with a central communication device. Network 108 may be a wide area network or a local area network. Network 108 may also include a plurality of networks.

A database 206 (as shown in Fig. 2) dedicated to an instrument 102 is a part (e.g., within the instrument) of an instrument 102.

The computing device is in communication with one or more other computing devices configured to receive indications of corresponding corrective actions determined to successfully resolve the issue at other instruments and provide the corresponding corrective actions to the computing device.

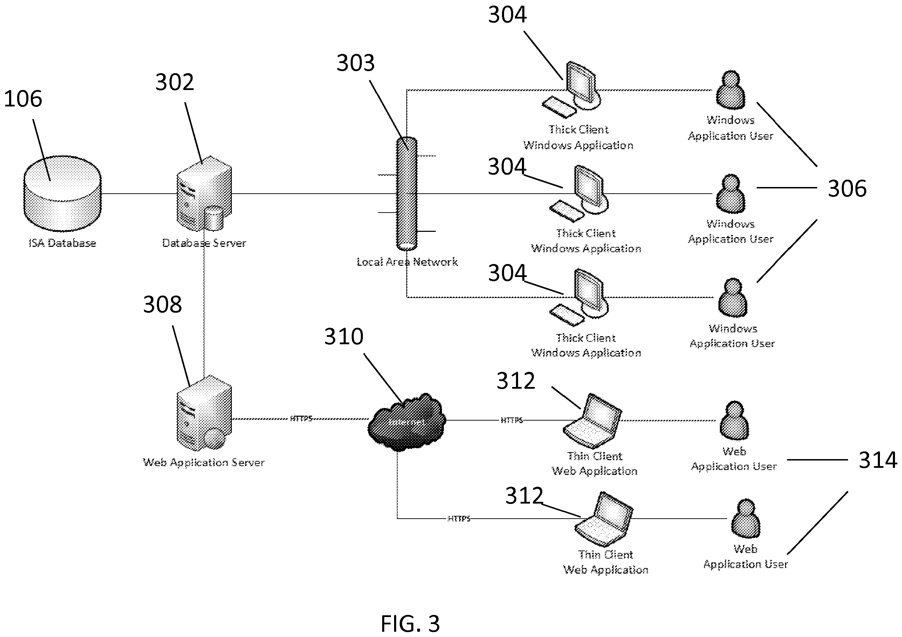

FIG. 3 is an illustration of an exemplary configuration of a communication architecture that may be used with embodiments disclosed herein. For example, as shown in FIG. 3, data base 106 may be in communication with a database server 302. The data base server 302 may be in communication with any number of clients, such as thick clients 304 (e.g., thick client windows application) via local area network 303. Thick clients 304 may provide users 306 with more features, graphics and choices making the applications more customizable. Processing for thick clients 304 may be performed locally on the user system (e.g., instrument 102), and may not rely heavily on a central processing server, such as database server 302. The server 302 may be accessed primarily for storage purposes. The data base server 302 may also be in communication with a web application server 308. The web application server 308 may be in communication with any number of clients, such as thin clients 312 (e.g., web application thin client) via a wide area network, such as the internet 310. In some embodiments, the communication system architecture may include any number of web application servers 308 in communication with clients via any number of wide area networks within the internet 310. Thin clients 312 may include little hardware and software and may rely more heavily on central processing server 302, allowing thin clients 312 to be centrally managed via software deployed on a central server, such as server 308, instead of each client 312 in order to provide services to web application users 314.

Exemplary methods of providing troubleshooting for an in vitro diagnostics instrument are now described. The method includes receiving, via a computing device, identification of one of a plurality of issues of the instrument, each of the plurality of issues associated with a portion of the instrument. For example, an issue may occur and be identified by processor 204.

In some embodiments, the instrument 102 may be self-aware of error conditions (e.g., a vessel with a sample is stuck on an internal transport system (from and to an analyzer)) and an event code or error code may be automatically generated (e.g., by a processor, such as processor 204, using instrument software) when an issue occurs. In other embodiments, the operator's selection of (e.g., selection of key words) via an interface, such as user interface 202, may provide a structured operator-defined issue for the instrument software to be translated into a non-event code or issue code, which may be rendered as an alpha-numeric code. Alpha-numeric codes may be received (e.g., by knowledge base content editor 104 and/or processor 204) when the ISA software client is launching. The alpha-numeric code may identify the issue and provide (e.g., via a link) an operator with the correct content in the user interface 202. The user interface 202 may prompt the operator to launch the ISA by selecting (e.g., via a button, a portion of a touch screen, or the like) on the interface 202. In some embodiments, content may be provided to an operator in a wizard style format.

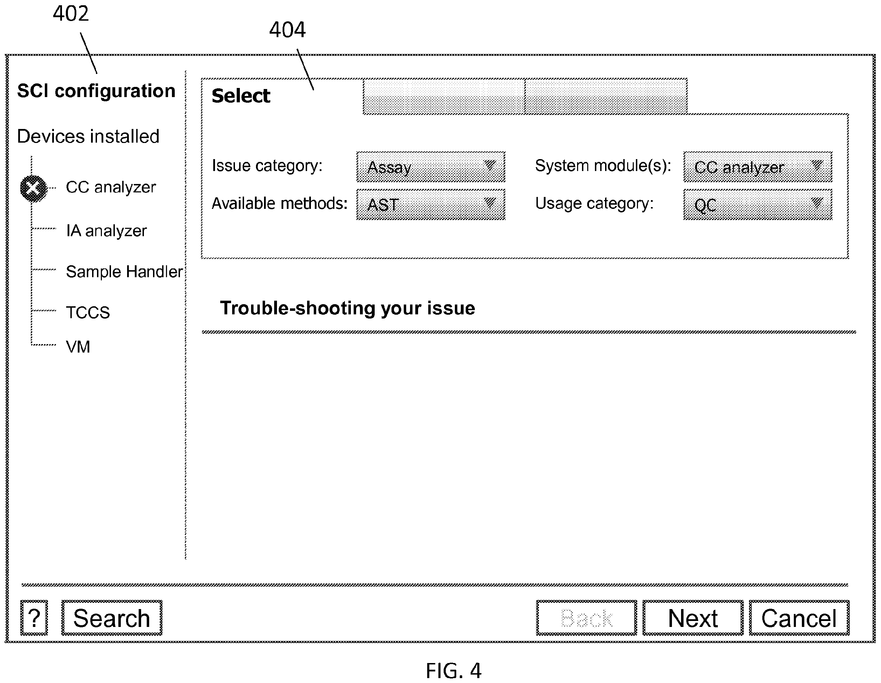

FIG. 4 is an exemplary screen shot illustrating an exemplary identification of one of a plurality of issues of the instrument 102. As shown in FIG. 4, the instrument configuration of installed devices (e.g., clinical chemical (CC) analyzer, an immunoassay (IA) analyzer and a sample handler) may be displayed in area 402.

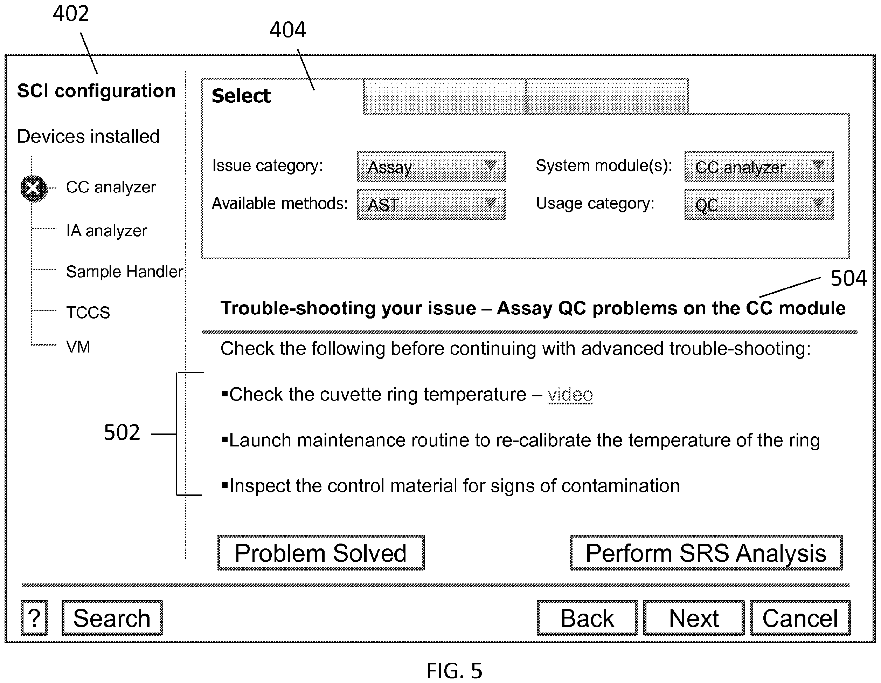

In the example shown in FIG. 5, the issue 504 is identified as "Assay QC problems on the CC module." A processor, such as processor 204, may automatically determine the issue 504 and generate an event code or error code. As shown in FIG. 5, the CC analyzer may be indicated (e.g., via "X" shown in FIG. 4) as having an event code. Selectable options corresponding to different categories may be automatically determined (e.g., via a processor, such as processor 204) and displayed using the event code, as shown in area 404. For example, the categories shown in FIG. 4 include: Issue category; Available methods; System modules; and Usage category. The selectable options for each category are automatically selected as: Issue Category: Assay; System Modules: CC Analyzer; Available Methods: AST; and Usage Category: quality control (QC). The categories and selectable options shown in FIG. 4 are exemplary. Embodiments may include categories and selectable options different than those shown in FIG. 4. In some embodiments, the operator may select the options for each category. For example, if no event code is available or only a generic event code is available, the operator may define a scope around the issue by triangulating the problem. When an issue is identified, an ordered list of one or more corrective actions may be provided to resolve the identified issue.

FIG. 5 is an exemplary screen shot illustrating a displayed ordered list 502 of corrective actions to resolve an identified issue 504. The ordered list 502 is stored in a database, such as database 206. The ordered list 502 is retrieved from database 206 and displayed via a user interface, such as user interface 202. In the example shown in FIG. 5, the issue 504 is identified as "Assay QC problems on the CC module."

The ISA knowledge base 106 may include the encoded customer service knowledge on how to troubleshoot an instrument issue (e.g., hardware, software, assay). In one embodiment, the problem statement and its corrective actions may be rendered in a decision-tree style format. For example, as shown in FIG. 5, the ordered list 502 of corrective actions is displayed in the following order: (1) Check the cuvette ring temperature - video; (2) Launch maintenance routine to re-calibrate the temperature of the ring; and (3) Inspect the control material for signs of contamination. The issue 504 displayed in FIG. 5 is merely exemplary. Embodiments may include issues different from the issue displayed in FIG. 5. The ordered list 502 of corrective actions displayed in FIG. 5 is also exemplary. Embodiments may include ordered lists different from the ordered list 502 displayed in FIG. 5 and may include any number of corrective actions. A video or other resource can be provided with each potential corrective action.

The ordered lists of corrective actions for solving issues is stored in databases (e.g., instrument database 206) prior to operation of instruments (e.g., at the time of instrument manufacturing).

Referring back to FIG. 5, the operator of the instrument 102 may review the ordered list 502 of corrective actions. The operator may check the temperature of a cuvette ring, launch a maintenance routine to re-calibrate the temperature of the cuvette ring and recalibrate the assay and perform a test using a third party QC material to verify if corrective actions were successful. If the issue is resolved by one or more of the corrective actions, an indication may be received that one or more corrective actions successfully resolved the issue 504.

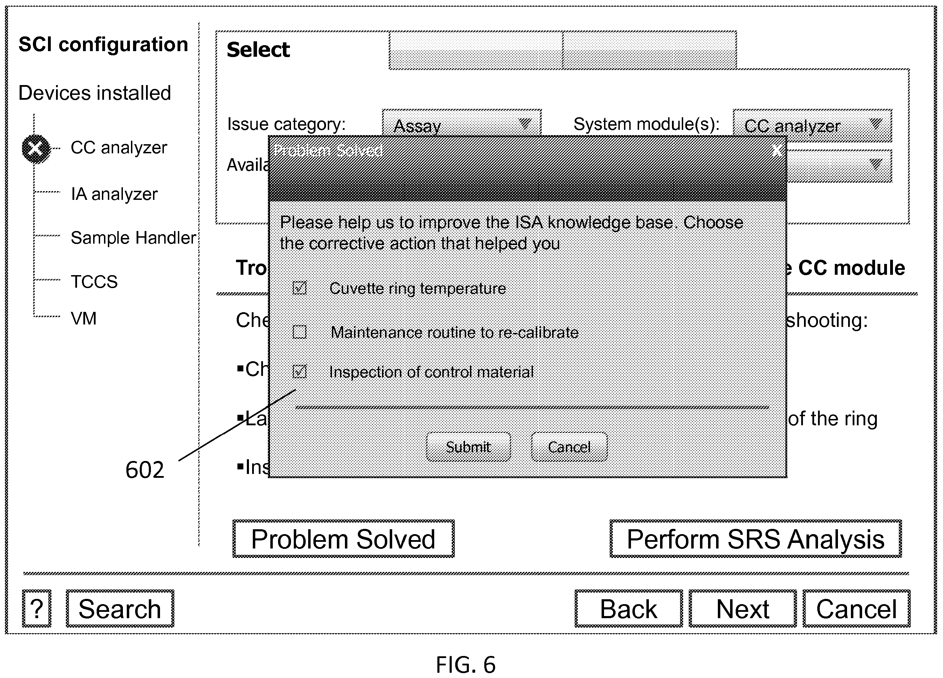

FIG. 6 is a screen shot illustrating an exemplary prompt area 602 prompting a user to indicate one or more of the corrective actions displayed in the ordered list 502 (shown in FIG. 5) to successfully resolve the identified issue 504 (shown in FIG. 5). The user interface 202 is used to prompt the operator and to receive the indication of one or more successful corrective actions. The indication is then be received by a computing device, such as processor 204. For example, as shown in FIG. 6, prompt area 602 prompts the user to choose which corrective actions (cuvette ring temperature, maintenance routine to recalibrate and inspection of control material) helped the operator. The corrective actions that were successfully used may be indicated (as shown by checkmarks) as cuvette ring temperature and inspection of control material. In another embodiment, one or more corrective actions may be automatically determined (e.g., via one or more sensors and one or more processors) to have successfully solved the issue. In some embodiments, the successful corrective actions may be stored locally at the instrument (e.g., database 206) and then sent to a second database, such as database 106 (shown in FIG. 1) in communication with a plurality of instruments 102 via one or more networks, such as network 108 (shown in FIG. 1). In other embodiments, the successful corrective actions may be sent to the second database without being stored at a local database.

In some embodiments, a report may be created that includes (i) the one or more corrective actions determined to successfully resolve the instrument issue and (ii) a navigation path indicating one or more steps used to provide the one or more corrective actions determined to successfully resolve the instrument issue. In some aspects, the report may be created by a local computing device (e.g., database 206) and then sent to a second database, such as database 106. In other embodiments, the report may be sent to the second database 106 without being stored at a local database. The successful corrective actions and/or the reports may be sent to the second database in equal time intervals (e.g., per hour, per day, per week), responsive to an event occurring, or on demand.

The second database 106 (e.g., database in communication with a plurality of instruments 102, such as ISA knowledge base 106), receives the successful corrective actions and/or reports and may analyze the corrective actions and/or reports. The analysis includes associating the corrective actions with corresponding issues and determining updated ordered lists of the corrective actions for each corresponding issue. The updated lists may then be sent to one or more instruments 102 in equal time intervals (e.g., per hour, per day, per week), responsive to an event occurring, or on demand.

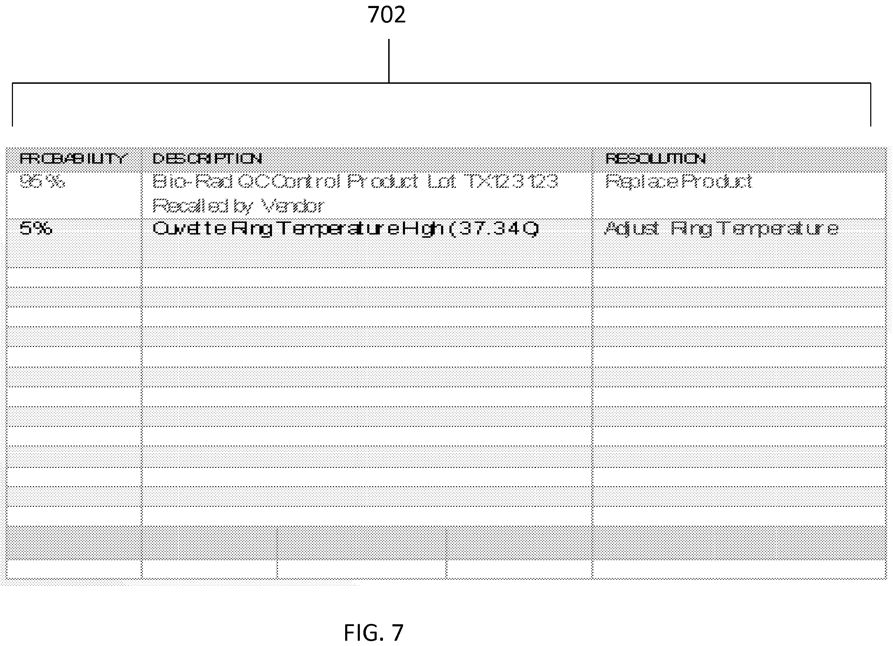

FIG. 7 is a screen shot illustrating an exemplary updated list 702 that is ordered based on a probability of the corrective actions to successfully solve the issue 504. As shown in FIG. 7, the first corrective action (Replace Product) of the updated list 702 includes a 95% probability of successfully solving the issue 504. The second corrective action (Adjust Ring Temperature) of the updated list 702 includes a 5% probability of successfully solving the issue 504. As shown, each corrective action may also include a description section having additional information.

The updated list shown in FIG. 7 is exemplary. Embodiments may include updated lists that are ordered based on: a success rate as indicated at each of the instruments 102; a probability of the corrective actions to successfully solve each corresponding issue; and; a priority. The updated list may include: the same corrective actions as the previous list but in a different order; one or more corrective actions that were not in the previous list; and one or more corrective actions removed from the previous list.

A central computing device, such as Knowledge Base Content Editor 104, may interface with any number of memory devices, such as read only memory (ROM), random access memory (RAM), and one or more optional non-transitory memory devices such as, for example, an external or internal DVD drive, a CD ROM drive, a hard drive, flash memory, a USB drive, or the like. The memory devices may be configured to include individual files and/or one or more databases for storing any software modules, instructions, or data.

Program instructions, software, or interactive modules for performing any of the functional steps associated with the processes as described above may be stored in the ROM and/or the RAM. Optionally, the program instructions may be stored on a tangible computer readable medium such as a compact disk, a digital disk, flash memory, a memory card, a USB drive, an optical disc storage medium, such as a Blu-ray^{™} disc, and/or other recording medium.

A display interface, such as a display included in user interface 202, may permit information from the ISA to be displayed on the display in audio, visual, graphic, and/or alphanumeric format. Communication with external devices may occur using various communication ports that may be attached to one or more communications networks, such as the Internet or a local area network, or directly to a portable computing device such as a notebook computer. The user interface 202 may allow for receipt of data from input devices such as a keyboard, a mouse, a joystick, a touch screen, a remote control, a pointing device, a video input device, an audio input device, and the like.

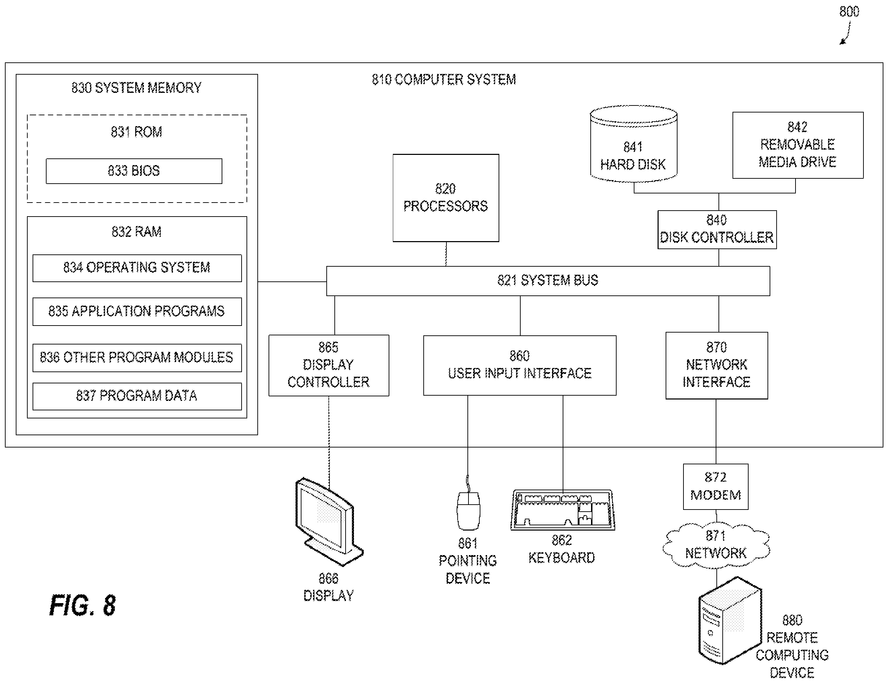

FIG. 8 illustrates an example of a computing environment 800 within which embodiments of the invention may be implemented. Computing environment 800 may be implemented as part of any component described herein. Computing environment 800 may include computer system 810, which is one example of a computing system upon which embodiments of the invention may be implemented. As shown in FIG. 8, the computer system 810 may include a communication mechanism such as a bus 821 or other communication mechanism for communicating information within the computer system 810. The system 810 further includes one or more processors 820 coupled with the bus 821 for processing the information. The processors 820 may include one or more CPUs, GPUs, or any other processor known in the art.

The computer system 810 also includes a system memory 830 coupled to the bus 821 for storing information and instructions to be executed by processors 820. The system memory 830 may include computer readable storage media in the form of volatile and/or nonvolatile memory, such as read only memory (ROM) 831 and/or random access memory (RAM) 832. The system memory RAM 832 may include other dynamic storage device(s) (e.g., dynamic RAM, static RAM, and synchronous DRAM). The system memory ROM 831 may include other static storage device(s) (e.g., programmable ROM, erasable PROM, and electrically erasable PROM). In addition, the system memory 830 may be used for storing temporary variables or other intermediate information during the execution of instructions by the processors 820. A basic input/output system 833 (BIOS) containing the basic routines that help to transfer information between elements within computer system 810, such as during start-up, may be stored in ROM 831. RAM 832 may contain data and/or program modules that are immediately accessible to and/or presently being operated on by the processors 820. System memory 830 may additionally include, for example, operating system 834, application programs 835, other program modules 836 and program data 837.

The computer system 810 also includes a disk controller 840 coupled to the bus 821 to control one or more storage devices for storing information and instructions, such as a magnetic hard disk 841 and a removable media drive 842 (e.g., floppy disk drive, compact disc drive, tape drive, and/or solid state drive). The storage devices may be added to the computer system 810 using an appropriate device interface (e.g., a small computer system interface (SCSI), integrated device electronics (IDE), Universal Serial Bus (USB), or FireWire).

The computer system 810 may also include a display controller 865 coupled to the bus 821 to control a display or monitor 866, such as a cathode ray tube (CRT) or liquid crystal display (LCD), for displaying information to a computer user. The computer system 810 includes a user input interface 860 and one or more input devices, such as a keyboard 862 and a pointing device 861, for interacting with a computer user and providing information to the processor 820. The pointing device 861, for example, may be a mouse, a trackball, or a pointing stick for communicating direction information and command selections to the processor 820 and for controlling cursor movement on the display 866. The display 866 may provide a touch screen interface which allows input to supplement or replace the communication of direction information and command selections by the pointing device 861.

The computer system 810 may perform a portion or all of the processing steps of embodiments of the invention in response to the processors 820 executing one or more sequences of one or more instructions contained in a memory, such as the system memory 830. Such instructions may be read into the system memory 830 from another computer readable medium, such as a hard disk 841 or a removable media drive 842. The hard disk 841 may contain one or more data stores and data files used by embodiments of the present invention. Data store contents and data files may be encrypted to improve security. The processors 820 may also be employed in a multi-processing arrangement to execute the one or more sequences of instructions contained in system memory 830. In alternative embodiments, hard-wired circuitry may be used in place of or in combination with software instructions. Thus, embodiments are not limited to any specific combination of hardware circuitry and software.

As stated above, the computer system 810 may include at least one computer readable medium or memory for holding instructions programmed according to embodiments of the invention and for containing data structures, tables, records, or other data described herein. The term "computer readable medium" as used herein refers to any non-transitory, tangible medium that participates in providing instructions to the processor 820 for execution. A computer readable medium may take many forms including, but not limited to, non-volatile media, volatile media, and transmission media. Non-limiting examples of non-volatile media include optical disks, solid state drives, magnetic disks, and magneto-optical disks, such as hard disk 841 or removable media drive 842. Non-limiting examples of volatile media include dynamic memory, such as system memory 830. Non-limiting examples of transmission media include coaxial cables, copper wire, and fiber optics, including the wires that make up the bus 821. Transmission media may also take the form of acoustic or light waves, such as those generated during radio wave and infrared data communications.

The computing environment 800 may further include the computer system 810 operating in a networked environment using logical connections to one or more remote computers, such as remote computer 880. Remote computer 880 may be a personal computer (laptop or desktop), a mobile device, a server, a router, a network PC, a peer device or other common network node, and typically includes many or all of the elements described above relative to computer 810. When used in a networking environment, computer 810 may include modem 872 for establishing communications over a network 871, such as the Internet. Modem 872 may be connected to system bus 821 via network interface 870, or via another appropriate mechanism.

Network 871 may be any network or system generally known in the art, including the Internet, an intranet, a local area network (LAN), a wide area network (WAN), a metropolitan area network (MAN), a direct connection or series of connections, a cellular telephone network, or any other network or medium capable of facilitating communication between computer system 810 and other computers (e.g., remote computing system 880). The network 871 may be wired, wireless or a combination thereof. Wired connections may be implemented using Ethernet, Universal Serial Bus (USB), RJ-11 or any other wired connection generally known in the art. Wireless connections may be implemented using Wi-Fi, WiMAX, and Bluetooth, infrared, cellular networks, satellite or any other wireless connection methodology generally known in the art. Additionally, several networks may work alone or in communication with each other to facilitate communication in the network 871.

A processor as used herein is a device for executing machine-readable instructions stored on a computer readable medium, for performing tasks and may comprise any one or combination of, hardware and firmware. A processor may also comprise memory storing machine-readable instructions executable for performing tasks. A processor acts upon information by manipulating, analyzing, modifying, converting or transmitting information for use by an executable procedure or an information device, and/or by routing the information to an output device. A processor may use or comprise the capabilities of a computer, controller or microprocessor, for example, and is conditioned using executable instructions to perform special purpose functions not performed by a general purpose computer. A processor may be coupled (electrically and/or as comprising executable components) with any other processor enabling interaction and/or communication therebetween. Computer program instructions may be loaded onto a computer, including without limitation, a general purpose computer or special purpose computer, or other programmable processing apparatus to produce a machine, such that the computer program instructions which execute on the computer or other programmable processing apparatus create means for implementing the functions specified in the block(s) of the flowchart(s). A user interface processor or generator is a known element comprising electronic circuitry or software or a combination of both for generating display elements or portions thereof. A user interface (UI) comprises one or more display elements enabling user interaction with a processor or other device.

An executable application, as used herein, comprises code or machine readable instructions for conditioning the processor to implement predetermined functions, such as those of an operating system, a context data acquisition system or other information processing system, for example, in response to user command or input. An executable procedure is a segment of code or machine readable instruction, sub-routine, or other distinct section of code or portion of an executable application for performing one or more particular processes. These processes may include receiving input data and/or parameters, performing operations on received input data and/or performing functions in response to received input parameters, and providing resulting output data and/or parameters. A graphical user interface (GUI), as used herein, comprises one or more display elements, generated by a display processor and enabling user interaction with a processor or other device and associated data acquisition and processing functions.

The UI also includes an executable procedure or executable application. The executable procedure or executable application conditions the display processor to generate signals representing the UI display images. These signals are supplied to a display device which displays the elements for viewing by the user. The executable procedure or executable application further receives signals from user input devices, such as a keyboard, mouse, light pen, touch screen or any other means allowing a user to provide data to a processor. The processor, under control of an executable procedure or executable application, manipulates the UI display elements in response to signals received from the input devices. In this way, the user interacts with the display elements using the input devices, enabling user interaction with the processor or other device. The functions and process steps herein may be performed automatically or wholly or partially in response to user command. An activity (including a step) performed automatically is performed in response to executable instruction or device operation without user direct initiation of the activity.

A workflow processor, as used herein, processes data to determine tasks to add to, or remove from, a task list or modifies tasks incorporated on, or for incorporation on, a task list, as for example specified in a program(s). A task list is a list of tasks for performance by a worker/user of a device or device or a combination of both. A workflow processor may or may not employ a workflow engine. A workflow engine, as used herein, is a processor executing in response to predetermined process definitions that implement processes responsive to events and event associated data. The workflow engine implements processes in sequence and/or concurrently, responsive to event associated data to determine tasks for performance by a device and or worker and for updating task lists of a device and a worker to include determined tasks. A process definition is definable by a user and comprises a sequence of process steps including one or more, of start, wait, decision and task allocation steps for performance by a device and or worker, for example. An event is an occurrence affecting operation of a process implemented using a process definition. The workflow engine includes a process definition function that allows users to define a process that is to be followed and may include an event monitor. A processor in the workflow engine tracks which processes are running, for which patients, physicians, and what step needs to be executed next, according to a process definition and may include a procedure for notifying physicians of a task to be performed.

## Claims

1. A method of providing troubleshooting for an in vitro diagnostics instrument (102) comprising a database (206), a user interface (202) and a computing device (204), the method comprising:
receiving, by the computing device (204), identification of one of a plurality of issues of the instrument (102), each of the plurality of issues associated with a portion of the instrument (102), wherein receiving identification of an issue comprises receiving one or more of (i) an issue code generated by software associated with the portion of the instrument, and (ii) a selection of parameters related to the portion of the instrument;
providing, via the user interface (202) in communication with the computing device (204), an ordered list of one or more corrective actions to resolve the issue, the ordered list being stored in the database (206) in communication with the computing device (204), wherein the ordered list of one or more corrective actions is stored in the database prior to operation of instrument, wherein the user interface comprises one or more display elements, wherein the user interface (202) is used to prompt an operator to indicate one or more of the corrective actions displayed in the ordered list to successfully resolve the identified issue and to receive the indication of one or more successful corrective actions ; and
receiving, by the computing device (204), an indication via the user interface (202) of the one or more corrective actions determined to successfully resolve the issue,
wherein the method further comprises:
providing, by the computing device (204) via a network (108), the one or more corrective actions determined to successfully resolve the issue to a second computing device (104), the second computing device (104) in communication with (i) the computing device (204) and (ii) one or more other computing devices configured to receive indications of corresponding corrective actions determined to successfully resolve one or more of the plurality of issues at other in vitro diagnostics instruments and provide the corresponding corrective actions to the second computing device (104); and
storing, at a second database (106) in communication with the second computing device (104), the one or more corrective actions determined to successfully resolve the issue at the in vitro diagnostics instrument (102) and the corresponding corrective actions determined to successfully resolve the one or more issues at the other in vitro diagnostics instruments.

2. The method of claim 1, wherein the selection of parameters comprises one or more of an issue category, a system module, an available method, and a usage category.

3. The method of claim 1, further comprising:
creating a report comprising (i) the one or more corrective actions determined to successfully resolve the issue and (ii) a navigation path indicating one or more steps used to provide the one or more corrective actions determined to successfully resolve the issue.

4. The method of claim 1, further comprising:
associating, by the second computing device, a plurality of corrective actions with corresponding issues; and
determining, by the second computing device, updated ordered lists of the one or more corrective actions for each corresponding issue based on at least one of: (i) a probability of the one or more corrective actions to successfully solve each corresponding issue; (ii) a success rate of the one or more corrective actions indicated at each of the instruments; and (iii) a priority; and
storing, at the second database, the ordering of the one or more corrective actions for solving each corresponding issue;
storing, at the database in communication with the computing device, one or more of the plurality of corrective actions and the corresponding issues associated with the one or more corrective actions;
receiving, from the second database, one of the determined updated ordered lists of the one or more corrective actions for solving the issue;
storing, at the database in communication with the computing device, the updated ordered list; and
providing, via the user interface in communication with the computing device, the updated ordered list.

5. The method of claim 4, wherein information provided to the second database and updates to the second database are provided through a second user interface of the second computing device.

6. The method of claim 1, wherein the computing device is in communication with one or more other computing devices configured to receive indications of corresponding corrective actions determined to successfully resolve the issue at other instruments and provide the corresponding corrective actions to the computing device.

7. The method of claim 1, wherein the issue is one of software-related, hardware-related, or assay-related.

8. A system (100) for providing troubleshooting for an in vitro diagnostics instrument (102), the system comprising the in vitro diagnostics instrument and the in vitro diagnostics instrument comprising a database (206), a user interface (202) and a computing device (204), the system, comprising the *in vitro* diagnostics instrument (102), comprising:
the database (206) configured to store a plurality of issues each associated with a portion of the instrument (102) and a plurality of corrective actions for resolving corresponding issues,
the user interface (202) comprising one or more display elements configured to: provide user interaction to identify an issue of the plurality of issues associated with a particular portion of the instrument (102); display an ordered list of one or more corrective actions for resolving the issue, prompt an operator to indicate one or more of the corrective actions displayed in the ordered list to successfully resolve the identified issue; and receive an indication of the one or more corrective actions that successfully resolved the issue, wherein the ordered list of one or more corrective actions is stored in the database prior to operation of the instrument; and
the computing device (204) in communication with the user interface (202), wherein the computing device (204) is configured to receive an identification of the issue and to determine the one or more corrective actions for resolving the issue,
the system further comprises
a central computing device (104) in communication with the computing device (204) and other computing devices (204) in communication with other instruments (102), the central computing device (104) configured to analyze the one or more corrective actions that successfully resolved the issue and other corrective actions that successfully resolved the issue at the other instruments (102) and provide an updated ordered list to the computing device (204) to be displayed at the user interface (202).

## Patentansprüche

1. Verfahren zum Bereitstellen einer Fehlersuche für ein In-vitro-Diagnoseinstrument (102), das eine Datenbank (206), eine Benutzeroberfläche (202) und eine Computervorrichtung (204) umfasst, wobei das Verfahren umfasst:
Empfangen, in der Computervorrichtung (204), einer Identifizierung von einem aus einer Vielzahl von Problemen des Instruments (102), wobei jedes aus der Vielzahl von Problemen, mit einem Teil des Instruments (102) assoziiert ist, wobei das Empfangen der Identifizierung eines Problems ein Empfangen umfasst von einem oder mehreren von: (i) einem Problemcode, der durch eine Software erzeugt wird, die mit dem Teil des Instruments assoziiert ist, und (ii) einer Auswahl von Parametern, die sich auf den Teil des Instruments beziehen;
Bereitstellen, über die Benutzeroberfläche (202), die in einer Kommunikation mit der Computervorrichtung (204) steht, einer geordneten Liste von einer oder mehreren korrigierenden Aktionen, um das Problem zu lösen, wobei die geordnete Liste in der Datenbank (206), die in einer Kommunikation mit der Computervorrichtung (204) steht, gespeichert wird, wobei die geordnete Liste der einen oder mehreren korrigierenden Aktionen in der Datenbank gespeichert wird, bevor das Instrument in Betrieb genommen wird, wobei die Benutzeroberfläche ein oder mehrere Anzeigeelemente umfasst, wobei die Benutzeroberfläche (202) verwendet wird, um einen Bediener aufzufordern, eine oder mehrere der korrigierenden Aktionen anzugeben, die in der geordneten Liste angezeigt werden, um das identifizierte Problem erfolgreich zu lösen und um die Indikation von einer oder mehreren erfolgreichen korrigierenden Aktionen zu empfangen; und
Empfangen, in der Computervorrichtung (204), einer Indikation über die Benutzeroberfläche (202) der einen oder mehreren korrigierenden Aktionen, die ermittelt wurde, um das Problem erfolgreich zu lösen,
wobei das Verfahren ferner umfasst:
Bereitstellen, durch die Computervorrichtung (204) über ein Netzwerk (108), der einen oder mehreren korrigierenden Aktionen, die ermittelt wurde, um das Problem erfolgreich zu lösen, für eine zweite Computervorrichtung (104), wobei die zweite Computervorrichtung (104) in einer Kommunikation mit (i) der Computervorrichtung (204) steht, und (ii) einer oder mehreren Computervorrichtungen steht, die konfiguriert sind, um Indikationen von entsprechenden korrigierenden Aktionen zu empfangen, die ermittelt wurde, um ein oder mehrere aus der Vielzahl von Problemen in anderen In-vitro-Diagnoseinstrument erfolgreich zu lösen und um die entsprechenden korrigierenden Aktionen für die zweite Computervorrichtung (104) bereitzustellen; und
Speichern, in einer zweiten Datenbank (106), die in einer Kommunikation mit der zweiten Computervorrichtung (104) steht, der einen oder mehreren korrigierenden Aktionen, die ermittelt wurde, um das Problem in dem In-vitro-Diagnoseinstrument (102) erfolgreich zu lösen, und der entsprechenden korrigierenden Aktionen, die ermittelt wurde, um das eine oder die mehreren Probleme in den anderen In-vitro-Diagnoseinstrumenten erfolgreich zu lösen.

2. Verfahren nach Anspruch 1, wobei die Auswahl von Parametern einen oder mehrere einer Problemkategorie, eines Steuermoduls, eines zur Verfügung stehenden Verfahrens und einer Nutzungskategorie umfasst.

3. Verfahren nach Anspruch 1, das ferner umfasst:
Erzeugen eines Berichts, der umfasst: (i) die eine oder mehreren korrigierenden Aktionen, die ermittelt wurde, um das Problem zu lösen, und (ii) einen Navigationspfad, der einen oder mehrere Schritte anzeigt, die verwendet werden, um die eine oder mehreren korrigierenden Aktionen bereitzustellen, die ermittelt wurde, um das Problem erfolgreich zu lösen.

4. Verfahren nach Anspruch 1, das ferner umfasst:
Assoziieren, durch die zweite Computervorrichtung, einer Vielzahl von korrigierenden Aktionen mit entsprechenden Problemen; und
Ermitteln, durch die zweite Computervorrichtung, von aktualisierten geordneten Listen der einen oder mehreren korrigierenden Aktionen für jedes entsprechende Problem basierend auf mindestens einem von: (i) einer Wahrscheinlichkeit der einen oder mehreren korrigierenden Aktionen, dass sie jedes entsprechende Problem erfolgreich lösen; (ii) einer Erfolgsrate der einen oder mehreren korrigierenden Aktionen, die in jedem der Instrumente angezeigt wird; und (iii) einer Priorität; und
Speichern, in der zweiten Datenbank, der Reihenfolge der einen oder mehreren korrigierenden Aktionen zum Lösen jedes entsprechenden Problems;
Speichern, in der Datenbank, die in einer Kommunikation mit der Computervorrichtung steht, einer oder mehreren aus der Vielzahl von korrigierenden Aktionen und der entsprechenden Probleme, die mit der einen oder den mehreren korrigierenden Aktionen assoziiert sind;
Empfangen, von der zweiten Datenbank, von einer der ermittelten aktualisierten geordneten Listen der einen oder mehreren korrigierenden Aktionen zum Lösen des Problems;
Speichern, in der Datenbank, die in einer Kommunikation mit der Computervorrichtung steht, der aktualisierten geordneten Liste; und
Bereitstellen, über die Benutzeroberfläche, die in einer Kommunikation mit der Computervorrichtung steht, der aktualisierten geordneten Liste.

5. Verfahren nach Anspruch 4, wobei die Informationen, die der zweiten Datenbank bereitgestellt werden, und Aktualisierungen für die zweite Datenbank durch eine zweite Benutzeroberfläche der zweiten Computervorrichtung bereitgestellt werden.

6. Verfahren nach Anspruch 1, wobei die Computervorrichtung in einer Kommunikation mit einer oder mehreren anderen Computervorrichtungen steht, die konfiguriert sind, um Indikationen von entsprechenden korrigierenden Aktionen zu empfangen, die ermittelt wurde, um das Problem in anderen Instrumenten erfolgreich zu lösen, und um die entsprechenden korrigierenden Aktionen für die Computervorrichtung bereitzustellen.

7. Verfahren nach Anspruch 1, wobei das Problem eines von softwarebezogen, hardwarebezogen oder untersuchungsbezogen ist.

8. System (100) zum Bereitstellen einer Fehlersuche für ein In-vitro-Diagnoseinstrument (102), wobei das System das In-vitro-Diagnoseinstrument umfasst, und wobei das In-vitro-Diagnoseinstrument eine Datenbank (206), eine Benutzeroberfläche (202) und eine Computervorrichtung (204) umfasst, wobei das System, welches das In-vitro-Diagnoseinstrument (102) umfasst, umfasst:
die Datenbank (206), die konfiguriert ist zum Speichern einer Vielzahl von Problemen, die jeweils mit einem Teil des Instruments (102) assoziiert sind, und einer Vielzahl von korrigierenden Aktionen zum Lösen der entsprechenden Probleme,
die Benutzeroberfläche (202), die ein oder mehrere Anzeigeelemente umfasst, die konfiguriert sind zum: Bereitstellen einer Benutzerinteraktion, um ein Problem aus der Vielzahl von Problemen zu identifizieren, das mit einem spezifischen Teil des Instruments (102) assoziiert ist; Anzeigen einer geordneten Liste von einer oder mehreren korrigierenden Aktionen zum Lösen des Problems; Auffordern eines Bedieners, um eine oder mehrere der korrigierenden Aktionen anzugeben, die in der geordneten Liste angezeigt werden, um das identifizierte Problem erfolgreich zu lösen; und Empfangen einer Indikation der einen oder mehreren korrigierenden Aktionen die das Problem erfolgreich gelöst haben, wobei die geordnete Liste der einen oder mehreren korrigierenden Aktionen in der Datenbank gespeichert wird, bevor das Instrument in Betrieb genommen wird; und
die Computervorrichtung (204), die in einer Kommunikation mit der Benutzeroberfläche (202) steht, wobei die Computervorrichtung (204) konfiguriert ist zum Empfangen einer Identifizierung des Problems und zum Ermitteln der einen oder mehreren korrigierenden Aktionen zum Lösen des Problems,
wobei das System ferner umfasst:
eine zentrale Computervorrichtung (104), die in einer Kommunikation mit der Computervorrichtung (204) steht, und andere Computervorrichtungen (204), die in einer Kommunikation mit anderen Instrumenten (102) stehen, wobei die zentrale Computervorrichtung (104) konfiguriert ist zum Analysieren der einen oder mehreren korrigierenden Aktionen, die das Problem erfolgreich gelöst haben, und anderer korrigierender Aktionen, die das Problem in den anderen Instrumenten (102) erfolgreich gelöst haben, und zum Bereitstellen einer aktualisierten geordneten Liste für die Computervorrichtung (204), damit sie auf der Benutzeroberfläche (202) angezeigt wird.

## Revendications

1. Procédé pour assurer un dépannage pour un instrument de diagnostic in vitro (102) comprenant une base de données (206), une interface utilisateur (202) et un dispositif informatique (204), le procédé comprenant les étapes consistant à :
recevoir, par le dispositif informatique (204), l'identification d'un de la pluralité de problèmes de l'instrument (102), chacun de la pluralité de problèmes étant associé à une partie de l'instrument (102), dans lequel la réception de l'identification d'un problème comprenant la réception d'un ou de plusieurs des éléments suivants : i) un code de problème généré par un logiciel associé à la partie de l'instrument, et ii) une sélection de paramètres liés à la partie de l'instrument ;
fournir, via l'interface utilisateur (202) en communication avec le dispositif informatique (204), une liste ordonnée d'une ou plusieurs actions correctives pour résoudre le problème, la liste ordonnée étant stockée dans la base de données (206) en communication avec le dispositif informatique (204), dans lequel la liste ordonnée d'une ou plusieurs actions correctives étant stockée dans la base de données avant l'utilisation de l'instrument, dans lequel l'interface utilisateur comprend un ou plusieurs éléments d'affichage, dans lequel l'interface utilisateur (202) est utilisée pour inviter un opérateur à indiquer une ou plusieurs actions correctives affichées dans la liste ordonnée pour résoudre avec succès le problème identifié et pour recevoir l'indication d'une ou plusieurs actions correctives réussies ; et
recevoir, par le dispositif informatique (204), une indication via l'interface utilisateur (202) de la ou des actions correctives déterminées pour résoudre avec succès le problème,
dans lequel le procédé comprend en outre les étapes consistant à :
fournir, par le dispositif informatique (204) via un réseau (108), la ou les actions correctives déterminées pour résoudre avec succès le problème à un second dispositif informatique (104), le second dispositif informatique (104) en communication avec (i) le dispositif informatique (204) et (ii) un ou plusieurs autres dispositifs informatiques configurés pour recevoir des indications d'actions correctives correspondantes déterminées pour résoudre avec succès un ou plusieurs de la pluralité de problèmes dans d'autres instruments de diagnostic in vitro et fournir les actions correctives correspondantes au second dispositif informatique (104) ; et
stocker, dans une seconde base de données (106) en communication avec le second dispositif informatique (104), la ou les actions correctives déterminées pour résoudre avec succès le problème au niveau de l'instrument de diagnostic in vitro (102) et les actions correctives correspondantes déterminées pour résoudre avec succès le ou les problèmes au niveau des autres instruments de diagnostic in vitro.

2. Procédé de la revendication 1, dans lequel la sélection des paramètres comprend un ou plusieurs éléments parmi une catégorie de problème, un module de système, un procédé disponible et une catégorie d'utilisation.

3. Procédé selon la revendication 1, comprenant en outre les étapes consistant à :
créer un rapport comprenant (i) la ou les actions correctives déterminées pour résoudre avec succès le problème et (ii) un chemin de navigation indiquant une ou plusieurs étapes utilisées pour fournir le ou les actions correctives déterminées pour résoudre avec succès le problème.

4. Procédé selon la revendication 1, comprenant en outre les étapes consistant à :
associer, par le second dispositif informatique, une pluralité d'actions correctives aux problèmes correspondants ; et
déterminer, par le second dispositif informatique, des listes ordonnées mises à jour de la ou des actions correctives pour chaque problème correspondant en fonction d'au moins un des éléments suivants : (i) une probabilité de la ou des actions correctives résolvent avec succès chaque problème correspondant ; (ii) un taux de réussite de la ou des actions correctives indiqué à chacun des instruments ; et (iii) une priorité ; et
stocker, dans la seconde base de données, l'ordre de la ou des actions correctives pour résoudre chaque problème correspondant ;
stocker, dans la base de données en communication avec le dispositif informatique, une ou plusieurs actions correctives parmi la pluralité d'actions correctives et les problèmes correspondants associés à la ou aux actions correctives ;
recevoir, à partir de la seconde base de données, une des listes ordonnées mises à jour déterminées d'une ou de plusieurs actions correctives pour résoudre le problème ;
stocker, dans la base de données en communication avec le dispositif informatique, la liste commandée mise à jour ; et
fournir, par l'intermédiaire de l'interface utilisateur en communication avec le dispositif informatique, la liste actualisée des commandes.

5. Procédé de la revendication 4, dans lequel les informations fournies à la seconde base de données et les mises à jour de la seconde base de données sont fournies par l'intermédiaire d'une seconde interface utilisateur du second dispositif informatique,

6. Procédé de la revendication 1, dans lequel le dispositif informatique est en communication avec un ou plusieurs autres dispositifs informatiques configurés pour recevoir des indications sur les actions correctives correspondantes déterminées pour résoudre avec succès le problème sur d'autres instruments et fournir les actions correctives correspondantes au dispositif informatique.

7. Procédé de la revendication 1, dans lequel le problème est lié au logiciel, au matériel ou à l'essai.

8. Système (100) pour assurer un dépannage d'un instrument de diagnostic in vitro (102), le système comprenant l'instrument de diagnostic in vitro et l'instrument de diagnostic in vitro comprenant une base de données (206), une interface utilisateur (202) et un dispositif informatique (204), le système, comprenant l'instrument de diagnostic *in vitro* (102), comprenant :
la base de données (206) configurée pour stocker une pluralité de problèmes associés chacun à une partie de l'instrument (102) et une pluralité d'actions correctives pour résoudre les problèmes correspondants,
l'interface utilisateur (202) comprend un ou plusieurs éléments d'affichage configurés pour : fournir une interaction avec l'utilisateur afin d'identifier un problème parmi la pluralité de problèmes associés à une partie particulière de l'instrument (102) ;
afficher une liste ordonnée d'une ou plusieurs actions correctives pour résoudre le problème, inviter un opérateur à indiquer une ou plusieurs actions correctives affichées dans la liste ordonnée pour résoudre avec succès le problème identifié ; et recevoir une indication de la ou des actions correctives qui ont résolu avec succès le problème, dans lequel la liste ordonnée d'une ou plusieurs actions correctives est stockée dans la base de données avant l'utilisation de l'instrument ; et
le dispositif informatique (204) en communication avec l'interface utilisateur (202), dans lequel le dispositif informatique (204) est configuré pour recevoir une identification du problème et pour déterminer une ou plusieurs actions correctives pour résoudre le problème,
le système comprend en outre
un dispositif informatique central (104) en communication avec le dispositif informatique (204) et d'autres dispositifs informatiques (204) en communication avec d'autres instruments (102), le dispositif informatique central (104) étant configuré pour analyser la ou les actions correctives qui ont permis de résoudre le problème et d'autres actions correctives qui ont permis de résoudre le problème au niveau des autres instruments (102), et pour fournir au dispositif informatique (204) une liste mise à jour des commandes à afficher sur l'interface utilisateur (202).
